# FASCICULE DE BREVET EUROPEEN

(11) **EP 2 443 082 B1**
(45) Date de publication et mention de la délivrance du brevet: **27.05.2015**
(21) Numéro de dépôt: 10724795.9
(22) Date de dépôt: 07.06.2010
(51) Int. Cl.: C07C 51/43, C07C 55/14

(54) **PROCEDE DE PRODUCTION DE CRISTAUX D'ACIDE ADIPIQUE.**
VERFAHREN ZUR HERSTELLUNG VON KRISTALLEN AUS ADIPINSÄURE
METHOD FOR PRODUCING CRYSTALS OF ADIPIC ACID

(30) Priorité: 16.06.2009 FR 0954014
(43) Date de publication de la demande: 25.04.2012
(73) Titulaire: Rhodia Opérations, 93306 Aubervilliers (FR)
(72) Inventeur: CARVIN, Philippe, F-69005 Lyon (FR); FOUCHER, Stéphanie, F-69330 Meyzieu (FR)
(74) Mandataire: Chatelan, Florence Anne
(86) Numéro de dépôt international: PCT/EP2010/057924
(87) Numéro de publication internationale: WO 2010/145961

(56) Documents cités:
- FR-A1- 2 795 721
- US-A- 5 471 001
- CHONG-HUI GU, AND DAVID J. W. GRANT: "Relationship Between Particle Size and Impurity Incorporation During Crystallization of (+)-Pseudoephedrine Hydrochloride, Acetaminophen, and Adipic Acid from Aqueous Solution" PHARMACEUTICAL RESEARCH, vol. 19, no. 7, juillet 2002 (2002-07), pages 1068-1071, XP002563839

## Description

La présente invention concerne un procédé de production de cristaux d'acide adipique.

Elle est relative, plus particulièrement, à un procédé de récupération de l'acide adipique sous forme de cristaux présentant une teneur en impuretés faible, obtenus par des étapes de cristallisation à partir notamment des milieux réactionnels de synthèse de l'acide adipique. Par teneur en impuretés, il faut comprendre la concentration en impuretés différentes de l'eau.

L'acide adipique est un grand produit chimique utilisé dans de nombreuses applications soit comme composé intermédiaire soit comme additif pour modifier les propriétés de certains produits.

Parmi ces applications, l'utilisation d'acide adipique comme monomère dans la production de polymères est la plus importante. Ainsi, l'acide adipique est un des monomères principaux pour la fabrication des polyamides, notamment du polyamide 6-6. Il est également utilisé pour la fabrication des polyesters, polyesters polyols et dans la production des polyuréthannes.

Dans ces applications et notamment dans les utilisations comme monomère pour la production de polyamides, l'acide adipique doit présenter un degré de pureté très élevé, c'est-à-dire une pureté d'un degré au moins équivalent à la pureté requise pour les composés actifs utilisés comme médicaments. En outre, les cristaux d'acide adipique doivent présenter certaines propriétés physiques, notamment de taille et de forme, pour obtenir une bonne coulabilité lors de l'alimentation de l'acide adipique dans les réacteurs ou stockeurs ainsi qu'une faible aptitude au mottage lors du stockage et du transport. Par ailleurs, la présence de cristaux de très faible taille, appelés généralement « fines », doit être minimisée pour des questions d'hygiène lors de la manipulation de l'acide adipique.

Dans ce contexte, la demande de brevet FR 2 795 721 décrit un procédé de fabrication de cristaux d'acide adipique consistant à traiter les cristaux obtenus après cristallisation selon un procédé comprenant les étapes de disperser lesdits cristaux dans un milieu liquide, d'agiter ledit milieu liquide pendant une durée déterminée pour obtenir la forme et l'état de surface des cristaux désirés, puis à séparer lesdits cristaux traités dudit milieu liquide.

Par ailleurs, le brevet US 5 471 001 décrit un procédé pour la cristallisation d'acide adipique selon lequel on soumet une liqueur mère aqueuse contenant de l'acide adipique dissout et des cristaux naissants d'acide adipique à une agitation ultrasonique de faible intensité tout en refroidissant la liqueur mère et/ou en diminuant le contenu en eau de la liqueur mère.

Enfin, Ch.-H. Gu et D. J. W. Grant décrivent dans Pharmaceutical Research, Vol. 19, n° 7 de juillet 2002, les relations existantes entre la taille des particules et l'incorporation d'impuretés durant la cristallisation d'acide adipique au départ d'une solution aqueuse.

L'acide adipique est, notamment, synthétisé par oxydation d'hydrocarbures, par exemple du cyclohexane, soit directement soit en deux étapes.

Le procédé le plus utilisé industriellement est le procédé d'oxydation du cyclohexane en deux étapes, comprenant une première étape d'oxydation du cyclohexane par l'oxygène en un mélange cyclohexanol/cyclohexanone, puis oxydation de ce mélange en acide adipique par l'acide nitrique. La première oxydation de cyclohexane en cyclohexanol/cyclohexanone peut être réalisée en une seule étape ou en deux phases en produisant dans une première phase l'hydroperoxyde de cyclohexyle en absence de catalyseur puis dans une deuxième phase la décomposition de l'hydroperoxyde de cyclohexyle en cyclohexanol/cyclohexanone, en présence d'un catalyseur. L'acide adipique est également produit par oxydation nitrique de cyclohexanol produit à partir du benzène par hydrogénation en cyclohexène et hydratation en cyclohexanol.

Dans ces procédés de synthèse, l'acide adipique est présent sous forme dissoute dans le milieu réactionnel qui comprend généralement de l'eau, de l'acide nitrique et de nombreux sous-produits de synthèse tels que des diacides différents de l'acide adipique comme les acides glutarique et succinique et des composés notamment métalliques provenant des catalyseurs utilisés et de la corrosion des appareillages.

L'acide adipique est récupéré de ce milieu réactionnel généralement par cristallisation après refroidissement et/ou concentration du milieu réactionnel. Cet acide adipique est purifié par cristallisations successives préférentiellement dans de l'eau, puis séché pour diminuer la teneur en humidité et ainsi éviter les problèmes de mottage ou agglomération pendant le stockage et le transport des cristaux.

Pour obtenir un degré de pureté élevé, les procédés utilisés comprennent de nombreuses étapes de cristallisation avec des phases de lavage et séchage importantes qui sont consommatrices d'énergie et de coûts d'investissement.

Toutefois, ces procédés, pour arriver à la qualité de pureté requise de l'acide adipique, comprennent de nombreuses étapes affectant l'économie du procédé et la qualité physique des cristaux par production de « fines », par exemple.

Il existe toujours une demande d'un procédé de purification de l'acide adipique plus performant permettant d'obtenir ce composé avec une pureté élevée et des cristaux présentant des propriétés physiques compatibles avec leur stockage, transport et manipulation.

A cet effet, l'invention propose un procédé de production de cristaux d'acide adipique par cristallisation permettant d'obtenir plus facilement et de manière plus économique des cristaux de pureté élevée et/ou présentant des propriétés physiques compatibles pour leur manipulation, transport et stockage.

L'invention propose un procédé de production de cristaux d'acide adipique à partir d'une solution d'acide adipique comprenant au moins une étape de purification par cristallisation et une étape de séparation et récupération des cristaux.

Selon l'invention, ce procédé de production des cristaux d'acide adipique tel que défini à la revendication 1, comprend une phase de broyage des cristaux avant l'étape de séparation et récupération des cristaux.

Le procédé de l'invention comprend généralement plusieurs étapes intermédiaires entre l'étape de cristallisation et l'étape de séparation et récupération comme, par exemple, des filtrations, repulpages, lavages. Au sens de la présente invention, on comprendra que « l'étape de purification par cristallisation et de séparation et récupération des cristaux d'acide adipique » englobe à la fois les phases de cristallisation et séparation et récupération ainsi que l'ensemble des phases intermédiaires.

Selon l'invention, l'étape de purification par cristallisation et de séparation et récupération des cristaux d'acide adipique comprend les étapes suivantes :
- une phase de cristallisation dans un cristallisoir comprenant éventuellement une boucle de circulation externe du milieu de cristallisation, par refroidissement et/ou par concentration d'une solution d'acide adipique,
- éventuellement une phase de séparation des cristaux formés, par exemple par filtration,
- éventuellement une phase de repulpage des cristaux dans un milieu liquide saturé en acide adipique,
- une phase de séparation et récupération des cristaux, avec éventuellement lavage des cristaux.

Par « étape (ou phase) de séparation et récupération des cristaux », il faut comprendre l'étape (ou phase) du procédé permettant de récupérer les cristaux d'acide adipique avant leur alimentation dans une nouvelle étape de purification ou une étape de séchage. Autrement dit, si le procédé met en oeuvre une seule étape de purification par cristallisation et de séparation et récupération des cristaux d'acide adipique, alors l'étape (ou phase) de séparation et récupération des cristaux sera la dernière étape avant séchage. Si le procédé met en oeuvre plusieurs étapes successives depurification par cristallisation et de séparation et récupération des cristaux d'acide adipique, alors « l'étape (ou phase) de séparation et récupération des cristaux » peut désigner la dernière étape avant séchage (pour la dernière purification) ou l'étape intermédiaire avant une nouvelle étape de purification par cristallisation et de séparation et récupération des cristaux d'acide adipique (pour les purifications précédant la dernière).

La phase de cristallisation peut être mise en oeuvre dans un cristallisoir ou réalisée dans plusieurs cristallisoirs montés en série.

Par « phase de repulpage » on entend au sens de la présente invention la mise en suspension ou dispersion des cristaux dans un milieu liquide, par exemple une solution saturée en acide adipique.

Par « phase de broyage des cristaux », il faut comprendre une phase au cours de laquelle les cristaux d'acide adipique sont cassés, plus ou moins fortement. Le broyage a pour effet de libérer ou de rendre déplaçables les impuretés piégées dans les cristaux comme, par exemple, le liquide de cristallisation ou la solution-mère de cristallisation incluse dans le cristal qui n'auraient pu être éliminées ou déplacées, par exemple, par lavage ou dans les phases de repulpage ou filtration (pour plus de clarté, le liquide de cristallisation ou solution-mère de cristallisation seront appelées eaux de cristallisation ou eaux-mères de cristallisation sans pour cela limiter la portée du brevet à l'utilisation de l'eau comme liquide utilisé pour réaliser la cristallisation de l'acide adipique). Comme ces eaux-mères ou eaux de cristallisation contiennent des sous produits ou impuretés présentes dans le milieu de cristallisation, le broyage des cristaux permet d'obtenir une concentration plus faible de ces impuretés en fin de procédé de purification de l'acide adipique.

Le broyage des cristaux est obtenu généralement par application d'une force mécanique sur les cristaux soit directement sur les cristaux humides ou sur les cristaux mis en suspension dans un liquide tel que de l'eau, avantageusement saturé en acide adipique. Les procédés convenables pour obtenir ce broyage sont tous les procédés usuels de broyage de produits solides tels que, par exemple, l'agitation, le concassage, l'écrasement.

Avantageusement, l'étape (ou phase) de séparation et récupération des cristaux peut être réalisée par tous procédés convenables, tels que, par exemple, décantation, filtration, centrifugation. Les procédés usuellement utilisés sont les filtres rotatifs ainsi que la centrifugation réalisée dans des appareils appelés « essoreuses ». Dans l'étape de séparation et récupération, les cristaux sont soumis à des lavages plus ou moins efficaces. Ces lavages ont notamment pour but d'éliminer les eaux mères présentes en surface des cristaux.

Le procédé de production des cristaux de l'acide adipique peut être réalisé en discontinu ou continu.

Selon un mode préféré de réalisation de l'invention, la solution d'acide adipique est une solution aqueuse d'acide adipique.

Selon l'invention, lorsque l'on met en oeuvre une seule étape de purification par cristallisation et de séparation et récupération des cristaux d'acide adipique, la phase de broyage, avant la phase de séparation et récupération des cristaux, est réalisée sur au moins l'un des milieux suivants :
o sur la suspension de cristaux contenus dans le cristallisoir et circulant dans l'éventuelle boucle externe au cristallisoir avec recyclage de cette suspension contenant les cristaux broyés dans le cristallisoir,
o sur les cristaux contenus dans le milieu de l'éventuelle phase de repulpage,
o sur les cristaux humides après l'éventuelle phase de repulpage,

Selon l'invention, lorsque l'on met en oeuvre au moins deux étapes successives de purification par cristallisation et de séparation et récupération des cristaux d'acide adipique, la phase de broyage, avant la phase de séparation et récupération des cristaux, est réalisée :
- à au moins l'une quelconque des étapes précédant la dernière étape de purification par cristallisation et de séparation et récupération des cristaux d'acide adipique, et/ou
- lors de la dernière étape de purification par cristallisation et de séparation et récupération des cristaux d'acide adipique sur au moins l'un des milieux suivants :
   o sur la suspension de cristaux contenus dans le cristallisoir et circulant dans l'éventuelle boucle externe au cristallisoir avec recyclage de cette suspension contenant les cristaux broyés dans le cristallisoir,
   o sur les cristaux contenus dans le milieu de l'éventuelle phase de repulpage,
   o sur les cristaux humides après l'éventuelle phase de repulpage.

Selon un mode de réalisation préféré de l'invention, le procédé de production de cristaux d'acide adipique comprend au moins deux étapes successives de purification par cristallisation.

La première étape de purification par cristallisation est réalisée à partir de l'acide adipique présent dans le milieu réactionnel de synthèse. Ainsi, le milieu réactionnel peut être éventuellement concentré par évaporation d'eau puis refroidi pour obtenir la cristallisation de l'acide adipique. Cette cristallisation peut être réalisée dans des cristallisoirs fonctionnant en continu ou en discontinu.

Les cristaux obtenus lors de la première phase cristallisation sont séparés des eaux-mères, par exemple, par filtration. Dans ce mode de réalisation, les cristaux sont ensuite dispersés dans un liquide tel que, par exemple, une solution aqueuse saturée en acide adipique dans une étape de repulpage avant d'être alimentés dans une étape de filtration/essorage pour éliminer le maximum d'eau, cette dernière étape de filtration/essorage correspondant à l'étape de séparation et récupération indiquée ci-dessus. Au cours de cette dernière étape de filtration/essorage ou préalablement à cette étape les cristaux sont lavés pour éliminer ou déplacer le maximum d'eaux mères ou de cristallisations présentes à la surface des cristaux et la remplacer par de l'eau ne contenant pas d'impuretés.

L'acide adipique obtenu dans cette première étape de purification est appelé « Acide adipique de qualité technique ». En effet, ces cristaux comprennent encore une quantité d'impuretés piégées dans le cristal et notamment des inclusions d'eaux mères. Ces inclusions ne peuvent être diminuées ou déplacées par des opérations de lavage ou repulpage.

Selon un mode de réalisation de l'invention, au cours de la première étape de purification par cristallisation et séparation et récupération des cristaux, les cristaux sont broyés par broyage des cristaux ou application d'une force mécanique (agitation forte) dans une suspension de cristaux, par exemple, dans la suspension des cristaux au cours de l'opération de repulpage. Il est également possible de réaliser ce broyage par disposition d'un moyen de broyage telle qu'une pompe, sur un circuit de circulation externe de la suspension de cristaux. Le broyage peut être réalisé dans une étape distincte avant alimentation des cristaux dans l'étape de repulpage.

Selon un autre mode de réalisation de l'invention, le cristallisoir comprend une boucle de circulation externe du milieu de cristallisation dans laquelle est disposé un dispositif de broyage. Dans ce mode de réalisation, le recyclage des cristaux broyés dans le cristallisoir permet de réaliser un ensemencement du milieu de cristallisation. Comme cela est bien connu dans le domaine de la cristallisation, l'ensemencement permet de contrôler et modifier la forme des cristaux obtenus.

Il est également possible de prévoir cette phase de broyage des cristaux sur une étape de traitement des cristaux récupérés après repulpage et essorage. Dans ce mode de réalisation, les cristaux repulpés, essorés une première fois, et éventuellement lavés sont dispersés dans un liquide saturé en acide adipique, avantageusement de l'eau saturée en acide adipique. Le broyage des cristaux est réalisé sur cette dispersion. Les cristaux d'acide adipique ainsi broyés sont récupérés dans une dernière étape de séparation récupération par tous procédés connus, par exemple, par filtration avec préférentiellement un lavage de ces cristaux. Ainsi, la phase de broyage des cristaux est réalisée sur les cristaux récupérés après une première phase de repulpage puis une première phase de séparation par filtration, lesdits cristaux broyés étant ensuite soumis à une nouvelle phase de repulpage avant d'être récupérés dans la phase de séparation et récupération.

Avantageusement, le broyage des cristaux d'acide adipique est mis en oeuvre dans la première étape de purification par cristallisation et séparation et récupération des cristaux pour obtenir des cristaux d'acide adipique de pureté déterminée. Toutefois, il est possible de prévoir une phase de broyage des cristaux dans les autres étapes suivantes de cristallisation, selon des modes de réalisation semblables à ceux décrits ci-dessus, notamment dans l'étape de cristallisation permettant d'obtenir un acide adipique de pureté élevée.

Avantageusement, le mode de réalisation du procédé de l'invention comprenant une étape de broyage dans la ou les premières étapes de purification, notamment pour produire de l'acide adipique de qualité technique, permet d'obtenir un acide adipique à un degré de pureté plus élevé qu'avec un procédé sans étape de broyage. Ainsi, la seconde ou dernière étape pour produire de l'acide adipique de pureté élevée, appelé usuellement « acide adipique de qualité purifié » pourra être un procédé de purification avec besoin d'efficacité moins élevée si on désire obtenir un acide adipique avec un degré de pureté équivalent à celui obtenu avec les procédés usuels. Egalement ce procédé de purification pourra permettre d'être moins traumatisant pour la forme et la taille des cristaux. Ainsi, le procédé de l'invention permet de produire de l'acide adipique soit avec une pureté très élevée ou avec une pureté équivalente à celle obtenue avec les procédés usuels mais avec notamment une teneur en fines plus basses permettant de diminuer l'aptitude au mottage des cristaux.

Pour déterminer la concentration en impuretés ou le degré de pureté de l'acide adipique, les concentrations en diacides glutarique et succinique sont mesurées ainsi que la concentration en ions nitrates.

D'autres détails et avantages de l'invention apparaitront plus clairement au vu des exemples donnés ci-dessous uniquement à titre illustratif et indicatif.

### EXEMPLE 1 : COMPARATIF

Un milieu réactionnel provenant du procédé d'oxydation nitrique d'un mélange cyclohexanol/cyclohexanone contient environ 24 % en poids d'acide adipique. Ce milieu comprend de l'eau, de l'acide nitrique, les différents composés issus de la réaction d'oxydation, des ions nitrates et métalliques provenant du catalyseur engagé et de la corrosion des appareillages.

Ce milieu est alimenté dans un cristallisoir. La cristallisation de l'acide adipique est obtenue par refroidissement à une température de l'ordre de 25 °C. Le solide cristallisé est séparé des eaux-mères de cristallisation par filtration.

Les cristaux ainsi récupérés sont mis en suspension dans de l'eau saturée en acide adipique à une température de 25 °C, dans une étape appelée "repulpage". Les cristaux ainsi traités sont alimentés dans un filtre. Les cristaux récupérés ont une taille moyenne de 437 µm.

Les cristaux ainsi filtrés sont lavés abondamment par alimentation d'eau saturée en acide adipique pour ainsi déplacer totalement les eaux de cristallisation présentes autour des cristaux. Cette méthode de lavage permet d'éliminer toutes impuretés non contenues dans les inclusions présentes dans les cristaux.

Les cristaux récupérés sont analysés pour déterminer la concentration en acide glutarique, acide succinique et ions nitrates.

Les teneurs en acide glutarique et succinique ainsi que celles en ions nitrates sont obtenues par mesure chromatographique en phase liquide après préparation de l'échantillon par dilution dans l'eau et comparaison avec un étalonnage externe dans les mêmes conditions. L'appareil utilisé comprend une colonne de type C18 ODS2, d'une détection conductimétrique ou spectroscopique ou UV notamment pour les ions nitrates. La solution éluante est une solution aqueuse diluée d'un acide minéral ou organique.

Les résultats obtenus sont :
- Ions nitrates : 723 ppm
- Acide succinique : 159 ppm
- Acide glutarique : 202 ppm

### EXEMPLE 2: INVENTION

L'exemple 1 est répété. Les cristaux présents dans l'étape de repulpage sont soumis à un broyage à l'aide d'un appareil ULTRATURAX disposé dans la dispersion de repulpage.

Les cristaux récupérés après filtration et lavages selon le processus décrit dans l'exemple 1 présentent les concentrations suivantes en impuretés.
- Ions nitrates : 283 ppm
- Acide succinique : 70 ppm
- Acide glutarique : 82 ppm

Les cristaux récupérés ont une dimension moyenne de 120 µm.

## Revendications

1. Procédé de production de cristaux d'acide adipique à partir d'une solution d'acide adipique, comprenant au moins une étape de purification par cristallisation et une étape de séparation et récupération des cristaux d'acide adipique, **caractérisé en ce que** les cristaux d'acide adipique sont soumis, avant l'étape de séparation et récupération, à une phase de broyage,
et **en ce que** l'étape de purification par cristallisation et de séparation et récupération des cristaux d'acide adipique comprend les étapes suivantes :
- une phase de cristallisation dans un cristallisoir comprenant éventuellement une boucle de circulation externe du milieu de cristallisation, par refroidissement et/ou par concentration d'une solution d'acide adipique,
- éventuellement une phase de séparation des cristaux formés,
- éventuellement une phase de repulpage des cristaux dans un milieu liquide saturé en acide adipique,
- une phase de séparation et récupération des cristaux, avec éventuellement lavage des cristaux,
et **en ce que**
- lorsque l'on met en oeuvre une seule étape de purification par cristallisation et de séparation et récupération des cristaux d'acide adipique, la phase de broyage, avant la phase de séparation et récupération des cristaux, est réalisée sur au moins l'un des milieux suivants :
o sur la suspension de cristaux contenus dans le cristallisoir et circulant dans l'éventuelle boucle externe au cristallisoir avec recyclage de cette suspension contenant les cristaux broyés dans le cristallisoir,
o sur les cristaux contenus dans le milieu de l'éventuelle phase de repulpage,
o sur les cristaux humides après l'éventuelle phase de repulpage,
- lorsque l'on met en oeuvre au moins deux étapes successives de purification par cristallisation et de séparation et récupération des cristaux d'acide adipique, la phase de broyage, avant la phase de séparation et récupération des cristaux, est réalisée à au moins l'une quelconque des étapes précédant la dernière étape de purification par cristallisation et de séparation et récupération des cristaux d'acide adipique et/ou lors de la dernière étape de par cristallisation et de séparation et récupération des cristaux d'acide adipique sur au moins l'un des milieux suivants :
o sur la suspension de cristaux contenus dans le cristallisoir et circulant dans l'éventuelle boucle externe au cristallisoir avec recyclage de cette suspension contenant les cristaux broyés dans le cristallisoir,
o sur les cristaux contenus dans le milieu de l'éventuelle phase de repulpage,
o sur les cristaux humides après l'éventuelle phase de repulpage.

2. Procédé selon la revendication 1, **caractérisé en ce que** la solution d'acide adipique est une solution aqueuse d'acide adipique.

3. Procédé selon la revendication 2, **caractérisé en ce que** l'acide adipique est synthétisé par oxydation nitrique d'un mélange cyclohexanol/cyclohexanone ou de cyclohexanol.

4. Procédé selon la revendication 2, **caractérisé en ce que** l'acide adipique est synthétisé par oxydation du cyclohexane par l'oxygène moléculaire.

5. Procédé selon l'une des revendications précédentes, **caractérisé en ce qu'**il comprend au moins deux étapes successives de purification par cristallisation de l'acide adipique.

6. Procédé selon la revendication 5 **caractérisé en ce que** la première étape de purification par cristallisation comprend :
• une phase de cristallisation dans un cristallisoir par refroidissement et/ou concentration d'une solution d'acide adipique,
• une phase de séparation des cristaux formés,
• une phase de repulpage des cristaux dans un milieu liquide saturé en acide adipique,
• une phase de séparation et récupération des cristaux, avec éventuellement lavage des cristaux,
• la phase de broyage étant mise en oeuvre sur les cristaux avant la phase de séparation et récupération des cristaux.

7. Procédé selon la revendication 6, **caractérisé en ce que** la phase de broyage des cristaux est réalisée sur les cristaux contenus dans le milieu de repulpage.

8. Procédé selon la revendication 6, **caractérisé en ce que** la phase de broyage des cristaux est réalisée sur les cristaux humides avant ou après la phase de repulpage.

9. Procédé selon la revendication 6, **caractérisé en ce que** le broyage des cristaux d'acide adipique est effectué sur la suspension de cristaux contenus dans le cristallisoir et circulant dans une boucle externe au cristallisoir avec recyclage de cette suspension contenant les cristaux broyés dans le cristallisoir.

10. Procédé selon la revendication 6, **caractérisé en ce que** la phase de broyage des cristaux est réalisée sur les cristaux récupérés après une première phase de repulpage puis une première phase de séparation par filtration, lesdits cristaux broyés étant ensuite soumis à une nouvelle phase de repulpage avant d'être récupérés dans la phase de séparation et récupération.

11. Procédé selon l'une des revendications précédentes, en ce que la séparation et récupération des cristaux est réalisée par filtration ou essorage.

12. Procédé selon l'une des revendications précédentes, **caractérisé en ce que** les cristaux séparés sont soumis à un ou plusieurs lavages.

13. Procédé selon l'une des revendications précédentes, **caractérisé en ce que** le broyage des cristaux est réalisée par agitation mécanique, concassage, écrasement.

## Patentansprüche

1. Verfahren zur Herstellung von Adipinsäurekristallen aus einer Adipinsäurelösung, umfassend mindestens einen Schritt der Reinigung durch Kristallisation und einen Schritt der Abtrennung und Gewinnung der Adipinsäurekristalle, **dadurch gekennzeichnet, dass** die Adipinsäurekristalle vor dem Schritt der Abtrennung und Gewinnung einer Zerkleinerungsphase unterworfen werden und dass der Schritt der Reinigung durch Kristallisation und der Schritt der Abtrennung und Gewinnung der Adipinsäurekristalle folgende Schritte umfasst:
- eine Phase der Kristallisation in einem Kristallisator, der gegebenenfalls eine Schleife zur externen Zirkulation des Kristallisationsmediums umfasst, durch Abkühlen und/oder durch Aufkonzentrieren einer Adipinsäurelösung,
- gegebenenfalls eine Phase der Abtrennung der gebildeten Kristalle,
- gegebenenfalls eine Phase der Repulpierung der Kristalle in einem mit Adipinsäure gesättigten flüssigen Medium,
- eine Phase der Abtrennung und Gewinnung der Kristalle, gegebenenfalls mit Waschen der Kristalle,
und dass
- dann, wenn ein einziger Schritt der Reinigung durch Kristallisation und der Abtrennung und Gewinnung der Adipinsäurekristalle durchgeführt wird, die Phase der Zerkleinerung vor der Phase der Abtrennung und Gewinnung der Kristalle an mindestens einem der folgenden Medien durchgeführt wird:
∘ an der Suspension von in dem Kristallisator enthaltenen und in der fakultativen kristallisatorexternen Schleife umlaufenden Kristallen mit Rückführung dieser die zerkleinerten Kristalle enthaltenden Suspension in den Kristallisator,
∘ an den Kristallen, die in dem Medium der fakultativen Repulpierungsphase enthalten sind,
∘ an den feuchten Kristallen nach der fakultativen Repulpierungsphase,
- dann, wenn mindestens zwei aufeinanderfolgende Schritte der Reinigung durch Kristallisation und der Abtrennung und Gewinnung der Adipinsäurekristalle durchgeführt werden, die Phase der Zerkleinerung vor der Phase der Abtrennung und Gewinnung der Kristalle in mindestens einem der dem letzten Schritt der Reinigung durch Kristallisation und der Abtrennung und Gewinnung der Adipinsäurekristalle vorhergehenden Schritte und/oder während des letzten Schritts der Reinigung durch Kristallisation und der Abtrennung und Gewinnung der Adipinsäurekristalle an mindestens einem der folgenden Medien durchgeführt wird:
∘ an der Suspension von in dem Kristallisator enthaltenen und in der fakultativen kristallisatorexternen Schleife umlaufenden Kristallen mit Rückführung dieser die zerkleinerten Kristalle enthaltenden Suspension in den Kristallisator,
∘ an den Kristallen, die in dem Medium der fakultativen Repulpierungsphase enthalten sind,
∘ an den feuchten Kristallen nach der fakultativen Repulpierungsphase.

2. Verfahren nach Anspruch 1, **dadurch gekennzeichnet, dass** es sich bei der Adipinsäurelösung um eine wässrige Adipinsäurelösung handelt.

3. Verfahren nach Anspruch 2, **dadurch gekennzeichnet, dass** die Adipinsäure durch Oxidation eines Gemischs von Cyclohexanol und Cyclohexanon oder von Cyclohexanol mit Salpetersäure synthetisierte wird.

4. Verfahren nach Anspruch 2, **dadurch gekennzeichnet, dass** die Adipinsäure durch Oxidation von Cyclohexan mit molekularem Sauerstoff synthetisiert wird.

5. Verfahren nach einem der vorhergehenden Ansprüche, **dadurch gekennzeichnet, dass** es mindestens zwei aufeinanderfolgende Schritte der Reinigung der Adipinsäure durch Kristallisation umfasst.

6. Verfahren nach Anspruch 5, **dadurch gekennzeichnet, dass** der erste Schritt der Reinigung durch Kristallisation Folgendes umfasst:
• eine Phase der Kristallisation in einem Kristallisator durch Abkühlen und/oder Aufkonzentrieren einer Adipinsäurelösung,
• eine Phase der Abtrennung der gebildeten Kristalle,
• eine Phase der Repulpierung der Kristalle in einem mit Adipinsäure gesättigten flüssigen Medium,
• eine Phase der Abtrennung und Gewinnung der Kristalle, gegebenenfalls mit Waschen der Kristalle,
• wobei die Phase der Zerkleinerung an den Kristallen vor der Phase der Abtrennung und Gewinnung der Kristalle durchgeführt wird.

7. Verfahren nach Anspruch 6, **dadurch gekennzeichnet, dass** die Phase der Zerkleinerung der Kristalle an den in dem Repulpierungsmedium enthaltenen Kristallen durchgeführt wird.

8. Verfahren nach Anspruch 6, **dadurch gekennzeichnet, dass** die Phase der Zerkleinerung der Kristalle an den feuchten Kristallen vor oder nach der Repulpierungsphase durchgeführt wird.

9. Verfahren nach Anspruch 6, **dadurch gekennzeichnet, dass** die Zerkleinerung der Adipinsäurekristalle an der Suspension von in dem Kristallisator enthaltenen und in einer kristallisatorexternen Schleife umlaufenden Kristallen mit Rückführung dieser die zerkleinerten Kristalle enthaltenden Suspension in den Kristallisator durchgeführt wird.

10. Verfahren nach Anspruch 6, **dadurch gekennzeichnet, dass** die Zerkleinerung der Kristalle an den nach einer ersten Repulpierungsphase und dann einer ersten Phase der Abtrennung durch Filtration gewonnenen Kristallen durchgeführt wird, wobei die zerkleinerten Kristalle dann einer erneuten Repulpierungsphase unterworfen und dann in der Phase der Abtrennung und Gewinnung gewonnen werden.

11. Verfahren nach einem der vorhergehenden Ansprüche, **dadurch gekennzeichnet, dass** die Abtrennung und Gewinnung der Kristalle durch Filtration oder Schleudern durchgeführt wird.

12. Verfahren nach einem der vorhergehenden Ansprüche, **dadurch gekennzeichnet, dass** die abgetrennten Kristalle einer oder mehreren Wäschen unterworfen werden.

13. Verfahren nach einem der vorhergehenden Ansprüche, **dadurch gekennzeichnet, dass** die Zerkleinerung der Kristalle durch mechanische Bewegung, Grobzerkleinerung, Brechen durchgeführt wird.

## Claims

1. Process for producing adipic acid crystals from a solution of adipic acid, comprising at least one step of purification by crystallization and one step of separation and recovery of the adipic acid crystals, **characterized in that** the adipic acid crystals are subjected, before the separation and recovery step, to a grinding phase, **in that** the step of purification by crystallization and of separation and recovery of the adipic acid crystals comprises the following steps:
- a crystallization phase in a crystallizer optionally comprising an external circulation loop of the crystallization medium, by cooling and/or by concentrating a solution of adipic acid;
- optionally a phase of separation of the crystals formed;
- optionally a phase of repulping the crystals in a liquid medium saturated with adipic acid;
- a phase of separation and recovery of the crystals, optionally with washing of the crystals;
and **in that**
- when a single step of purification by crystallization and of separation and recovery of the adipic acid crystals is used, the grinding phase, before the phase of separation and recovery of the crystals, is carried out on at least one of the following media:
∘ on the suspension of crystals contained in the crystallizer and circulating in the optional loop external to the crystallizer with recycling of this suspension containing the ground crystals into the crystallizer;
∘ on the crystals contained in the medium of the optional repulping phase;
∘ on the wet crystals after the optional repulping phase;
- when at least two successive steps of purification by crystallization and of separation and recovery of the adipic acid crystals are carried out, the grinding phase, before the phase of separation and recovery of the crystals, is carried out, at at least any one of the steps preceding the last step of purification by crystallization and of separation and recovery of the adipic acid crystals and/or during the last step of purification by crystallization and of separation and recovery of the adipic acid crystals, on at least one of the following media:
∘ on the suspension of crystals contained in the crystallizer and circulating in the optional loop external to the crystallizer with recycling of this suspension containing the ground crystals into the crystallizer;
∘ on the crystals contained in the medium of the optional repulping phase;
∘ on the wet crystals after the optional repulping phase.

2. Process according to Claim 1, **characterized in that** the solution of adipic acid is an aqueous solution of adipic acid.

3. Process according to Claim 2, **characterized in that** the adipic acid is synthesized by nitric acid oxidation of a cyclohexanol/cyclohexanone mixture or of cyclohexanol.

4. Process according to Claim 2, **characterized in that** the adipic acid is synthesized by oxidation of cyclohexane with molecular oxygen.

5. Process according to one of the preceding claims, **characterized in that** it comprises at least two successive steps of purification by crystallization of the adipic acid.

6. Process according to Claim 5, **characterized in that** the first step of purification by crystallization comprises:
• a phase of crystallization in a crystallizer by cooling and/or concentrating a solution of adipic acid;
• a phase of separation of the crystals formed;
• a phase of repulping of the crystals in a liquid medium saturated with adipic acid;
• a phase of separation and recovery of the crystals, with optional washing of the crystals; and
• the grinding phase being carried out on the crystals before the phase of separation and recovery of the crystals.

7. Process according to Claim 6, **characterized in that** the grinding phase of the crystals is carried out on the crystals contained in the repulping medium.

8. Process according to Claim 6, **characterized in that** the grinding phase of the crystals is carried out on the wet crystals before or after the repulping phase.

9. Process according to Claim 6, **characterized in that** the grinding of the adipic acid crystals is carried out on the suspension of crystals contained in the crystallizer and circulating in a loop external to the crystallizer with recycling of this suspension containing the ground crystals into the crystallizer.

10. Process according to Claim 6, **characterized in that** the grinding phase of the crystals is carried out on the crystals recovered after a first repulping phase then a first phase of separation by filtration, said ground crystals then being subjected to a new repulping phase before being recovered in the separation and recovery phase.

11. Process according to one of the preceding claims, **characterized in that** the separation and recovery of the crystals is carried out by filtration or centrifugal drying.

12. Process according to one of the preceding claims, **characterized in that** the separated crystals are subjected to one or more washing operations.

13. Process according to one of the preceding claims, **characterized in that** the grinding of the crystals is carried out by mechanical agitation, pounding and crushing.
